# EUROPEAN PATENT APPLICATION

(11) **EP 3 859 739 A1**
(43) Date of publication of application: **04.08.2021**
(21) Application number: 21154780.7
(22) Date of filing: 02.02.2021
(51) Int. Cl.: G16B 20/20

(54) **PHENOTYPE INFERENCE BASED ON INCOMPLETE GENETIC DATA**

(30) Priority: 03.02.2020 RU 2020104947
(71) Applicant: Atlas Biomed Group Limited, London E1W 1DD (GB)
(72) Inventor: Nikogosov, Dimitri, 119146 Moscow (RU); Danilov, Kirill, 427621 Glazov (RU)
(74) Representative: Papula Oy

(57) **Abstract**

The invention relates to means of a user's phenotype inference in the absence of complete genetic information and providing recommendations based on the inferred phenotypes. The invention solves the problem of accurately inferring a user's phenotype on the basis of the user's incomplete genetic data expressed in a lack of information about the genotype for at least one position or identifier of a genetic variation. The invention relates to a method, a system and a computer-readable data medium that allows obtaining a user's genetic information with genetic variations in the form of position-genotype pairs, selecting genetic variations that correspond to data on known haplotypes of one or more genes, from the user's genetic information, obtaining all possible diplotypes from known haplotypes for the genes, evaluating each obtained diplotype for compliance with the obtained genetic information of the user, forming a set of selected diplotypes on the basis of the calculated total number of mismatches, mapping possible diplotypes for said genes to a phenotype, inferring the user's phenotype on the basis of the resulting mapping from diplotypes to phenotypes.

## Description

The present invention generally relates to computing systems as well as to their application in genetics and particularly relates to systems and methods of a user's phenotype inference with the use of genetic information.

### BACKGROUND OF THE INVENTION

The concept of genotype includes the totality of all genes of an organism, which have been inherited from its parents and directly influence the phenotype expression, i.e. the development of particular external and internal features (traits) of the organism. Also, this term is often used in a restricted sense, and in this case, genotype means a combination of gene alleles controlling the expression of a trait.

DNA nucleotide sequence variation is the diversity of variants for a DNA sequence region, which are represented in a population. Variants may be introduced, for example, by replacing one nucleotide with another (single nucleotide variation), changing the order of nucleotides, inserting or deleting one or more nucleotides (insertion-deletion variation), changing the number of repetitive DNA fragments, etc.

A nucleotide sequence variation in a DNA molecule region encoding a gene may introduce differences in the encoded protein, for example, by changing one or more amino acids, which in turn may influence the structure and/or function of this protein. A nucleotide sequence variation in a DNA molecule region regulating the activity of genes may change the amount of produced protein without changing its properties, which may also influence the phenotype. It is also important that there can be several hundred known possible variations for a single gene and therefore thousands possible combinations of such variations for a diploid chromosome set, so special algorithms are needed for automatic analysis of genotyping data.

There are several genotype-based haplotype recovery algorithms that have been developed and implemented over the past 30 years. The significant improvement of sequencing technologies allowed fundamentally new approaches for inferring individual haplotypes from next-generation sequencing data. However, most of the known algorithms are aimed at recovering a whole-genome haplotype or processing large sequences, when the main objective is to optimize the calculations. Similar algorithms use heuristic or graph approaches for data on raw reads, and more than one nucleotide sequence variation may fall within the boundaries of each read, *(review example:* Rhee, J. K., Li, H., Joung, J. G., Hwang, K. B., Zhang, B. T., & Shin, S. Y. (2016) Survey of computational haplotype determination methods for single individual. Genes & Genomics, 38(1), 1-12*).*

Such algorithms are not suitable for inferring haplotypes when it comes to a single gene or a group of genes. The inference of haplotypes for a gene is based on single nucleotide variations (SNVs) and insertions-deletions (INDELs) and requires fundamentally different approaches.

### SUMMARY OF THE INVENTION

The disclosed invention offers an opportunity to solve a number of problems related to inferring a user's phenotype and to provide the user with appropriate personalized recommendations, as will be described in detail below.

Thus, the technical result, at which the present invention is aimed, is to enable accurate inference of a user's phenotype on the basis of his/her incomplete genetic data expressed in lack of information about the genotype for at least one position and to provide the user with personalized recommendations, taking into account the user's inferred phenotype.

The above technical result is achieved through the present invention thanks to the fact that in one of the alternative embodiments, the disclosed invention is a method of a user's phenotype inference in the absence of complete genetic information by a data processing device, the method comprises: obtaining a user's genetic information with genetic variations in the form of position-genotype pairs and/or identifier-genotype pairs, and the incompleteness of the user's genetic information is reflected in the absence of a genotype for at least one position and/or identifier of a genetic variation, selecting genetic variations that correspond to data on known haplotypes of at least one gene, which are contained in a data storage, from the user's genetic information, thus obtaining at least one set of genetic variations U from the user's genetic information, obtaining all possible diplotypes from known haplotypes for said one or more genes, evaluating each obtained diplotype for compliance with the obtained genetic information of the user by calculating the number of mismatches between the diplotype and the set of variations U, forming at least one set of selected diplotypes D on the basis of the calculated total number of mismatches for each obtained diplotype, mapping at least one possible diplotype for said one or more genes to a phenotype from said data storage, and inferring the user's phenotype on the basis of the resulting mapping from diplotypes to phenotypes, by using the set D.

In an alternative embodiment, the invention is the method of a user's phenotype inference, wherein the data storage additionally stores information about the frequency of known haplotypes.

In an alternative embodiment, the invention is the method of a user's phenotype inference, wherein the data storage stores a description of the molecular effects of each known haplotype for said one or more genes.

In an alternative embodiment, the invention is the method of a user's phenotype inference, wherein molecular effects of at least one diplotype for said one or more genes are determined based on a description of the molecular effects of at least one haplotype constituting this diplotype, and a mapping from at least one diplotype to a phenotype is calculated based on the description of the molecular effects of this diplotype.

In an alternative embodiment, the invention is the method of a user's phenotype inference, wherein all possible diplotypes may be used for the formation of the set D if no variant of the diplotype with a complete match with the set of variations U is found when evaluating each obtained diplotype.

In an alternative embodiment, the invention is the method of a user's phenotype inference, wherein at least one threshold value applies to the number of mismatches, which excludes one or more diplotypes from further consideration when evaluating each obtained diplotype.

In an alternative embodiment, the invention is the method of a user's phenotype inference, wherein the median value of the distribution of the number of mismatches may be selected as the said threshold value.

In an alternative embodiment, the invention is the method of a user's phenotype inference, wherein the threshold value may be determined by adding the minimum number of mismatches from the calculated set of mismatches and the calculated number of genetic variations with an unknown genotype in the user's genetic information.

In an alternative embodiment, the invention is the method of a user's phenotype inference, wherein for each diplotype from the set D, a probability value is calculated based on data on its haplotype frequency, and the probability value is equal to the product of the frequencies of haplotypes constituting the diplotype for homozygous diplotypes and for phased heterozygous diplotypes or twice the product of the frequencies of haplotypes constituting the diplotype for unphased heterozygous diplotypes or the value of the frequency of a constituent haplotype for hemizygous diplotypes.

In an alternative embodiment, the invention is the method of a user's phenotype inference, which includes adding genetic variations of known haplotypes of at least one gene to said at least one set of variations U of the user's genetic information, in case there is no genetic information of the user for these variations.

In an alternative embodiment, the invention is the method of a user's phenotype inference, which includes calculating the probability of each phenotype for the user.

In an alternative embodiment, the invention is the method of a user's phenotype inference, wherein at least one surjective mapping that completely maps a larger set of diplotypes to a smaller set of phenotypes is used as a mapping, and the probability of each phenotype for the user may be calculated as the sum of the previously obtained probabilities of the corresponding diplotypes from the set D.

In an alternative embodiment, the invention is the method of a user's phenotype inference, which includes forming one or more recommendations for the user, taking into account the inferred phenotype.

In an alternative embodiment, the invention is the method of a user's phenotype inference, wherein forming one or more recommendations for the user includes at least one of: a recommendation to change the drug dosage or a recommendation to refuse drug administration because of the inefficacy and/or toxicity of said drug for the user's previously inferred phenotype or a recommendation to use alternative drugs that are more effective and/or safer for the user or a recommendation to dose the drug in accordance with the instructions for medical use and taking into account the inferred phenotype.

In an alternative embodiment, the invention is the method of a user's phenotype inference, wherein at least one gene belongs to a group of genes of drug-metabolizing enzymes or genes of drug transporters or genes of human leukocyte antigen or genes of drug targets.

In an alternative embodiment, the invention is the method of a user's phenotype inference, wherein when calculating the number of mismatches between the diplotype and the set of variations U, the determined number of mismatches is zero and implies a complete match between them.

In an alternative embodiment, the invention is the method of a user's phenotype inference, wherein all possible diplotypes are obtained by extracting data on these diplotypes from a data storage.

The above result is also achieved thanks to the fact that in another alternative embodiment, the claimed invention is a system for inferring a user's phenotype in the absence of complete genetic information, which comprises a data processing device and a memory operatively linked to the processing device, and this system is configured for obtaining a user's genetic information with genetic variations in the form of position-genotype pairs and/or identifier-genotype pairs, and the incompleteness of the user's genetic information is reflected in the absence of a genotype for at least one position and/or identifier of a genetic variation, selecting genetic variations that correspond to data on known haplotypes of at least one gene, which are contained in a data storage, from the user's genetic information, thus obtaining at least one set of genetic variations U from the user's genetic information, obtaining all possible diplotypes from known haplotypes for said one or more genes, evaluating each obtained diplotype for compliance with the obtained genetic information of the user by calculating the number of mismatches between the diplotype and the set of variations U, forming at least one set of selected diplotypes D on the basis of the calculated total number of mismatches, mapping at least one possible diplotype for said one or more genes to a phenotype from said data storage, and inferring the user's phenotype on the basis of the resulting mapping from diplotypes to phenotypes, by using the set D.

The above result is also achieved thanks to the fact that in another alternative embodiment, the claimed invention is a computer-readable data medium that is configured to be read by a data processing device and stores commands to be executed by a data processing device for inferring a user's phenotype in the absence of complete genetic information, and said commands, when executed by the data processing device, induce it to execute obtaining a user's genetic information with genetic variations in the form of position-genotype pairs and/or identifier-genotype pairs, and the incompleteness of the user's genetic information is reflected in the absence of a genotype for at least one position and/or identifier of a genetic variation, selecting genetic variations that correspond to data on known haplotypes of at least one gene, which are contained in a data storage, from the user's genetic information, thus obtaining at least one set of genetic variations U from the user's genetic information, obtaining all possible diplotypes from known haplotypes for said one or more genes, evaluating each obtained diplotype for compliance with the obtained genetic information of the user by calculating the number of mismatches between the diplotype and the set of variations U, forming at least one set of selected diplotypes D on the basis of the calculated total number of mismatches, mapping at least one possible diplotype for said one or more genes to a phenotype from said data storage, and inferring the user's phenotype on the basis of the resulting mapping from diplotypes to phenotypes, by using the set D.

It should be obvious to a person skilled in the art that the claimed invention is not limited to the above embodiments, as detailed below in the description.

### BRIEF DESCRIPTION OF THE DRAWINGS

The features and advantages of this technical disclosure will be evident from the following detailed description and attached drawings where:

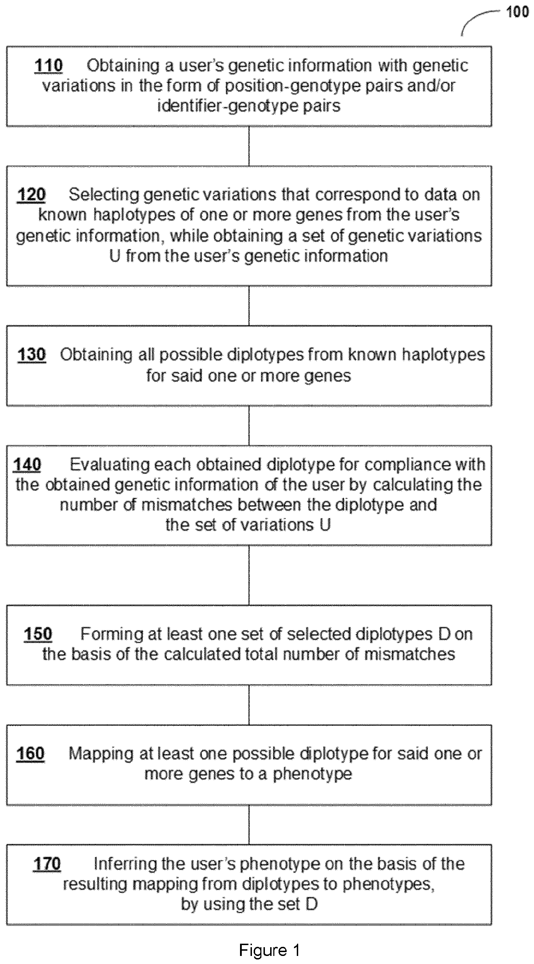

Figure 1 is an example of an implementation of the method of phenotype inference in the absence of a user's complete genetic information in the form of a flow diagram.
Figure 2 is a diagram for obtaining all possible diplotypes and calculating the total number of mismatches between each variant of these diplotypes and individual data of genotyping.
Figure 3 is a diagram for calculating the normalized value of diplotype probability.
Figure 4A schematically shows inferring a user's phenotype by means of a surjective mapping, provided that data on his/her genotypes are complete.
Figure 4B schematically shows inferring a user's phenotype by means of a surjective mapping through calculating probability values for the presence of a particular diplotype.
Figure 4C schematically shows inferring a user's phenotype by means of a composition of surjective mappings through intermediately mapping diplotypes to molecular effects.
Figure 5 is a diagram illustrating the proposed method of phenotype inference by using a composition of surjective mappings, as exemplified by clopidogrel metabolism. The dashed line shows elements of the codomain, to which genotype data were not mapped.
Figure 6A is a diagram illustrating the proposed method of phenotype inference and recommendation formation by using a composition of surjective mappings, as exemplified by blood vitamin D levels.
Figure 6B is a diagram illustrating the proposed method of phenotype inference and recommendation formation by using a composition of surjective mappings, as exemplified by blood vitamin D levels for another set of genotypes.
Figure 7 is a component diagram for an example of a computing system that may be used to implement the method described herein of a user's phenotype inference in the absence of complete genetic information.

### INVENTION DISCLOSURE

The technical disclosure described below may be implemented on a computer or another data processing device in the form of an automated system or a computer-readable medium containing commands for the embodiment of the above-mentioned method.

The technical disclosure described below may be implemented in the form of a distributed computer system, the components of which are cloud or local servers.

Some parts of the technical disclosure described herein are presented in terms of algorithms and other representations of operations with data bits or binary digital signals in computer memory. It is appreciated that the definitions or conclusions mentioned in this description can be implemented with the use of artificial intelligence techniques. More specifically, the terms "processing," "calculation," "determination," "establishment," "analysis," "identification," "verification" etc. may relate to operations and/or processes of a computer, a computing platform, a computer system or another electronic device that manipulates and/or transforms data represented as physical (e.g. electronic) quantities within the computer registers and/or memory devices into other data similarly represented as physical quantities within registers and/or storage devices of a computer or another storage medium that can store commands for executing the undermentioned methods, operations and/or processes.

The terms and their definitions used in the description of the technical disclosure will be considered in detail below.

In this description, a system means a computer system or an automated system (AS), an electronic computing machine (ECM), numeric control (NC), a programmable logic controller (PLC), a computer-assisted command system, a mobile device and any other devices capable of performing a specified, clearly defined sequence of calculation operations (actions, commands).

A command-processing device means an electronic unit or an integrated circuit (microprocessor) which executes computer-based commands (programs). The command-processing device reads and executes computer-based commands (programs) from one or more data storage devices. A data storage device may represent hard disk drives (HDD), flash memory, memory cards, read-only memory (ROM), solid-state drives (SSD), optical disc drives (ODD), cloud storages, etc.

A program is a sequence of commands to be executed by a computer control device or a command-processing device.

DNA sequencing is determining the sequence of nucleotides in a DNA molecule. This can be understood as both amplicon sequencing (reading sequences of extracted DNA fragments from a PCR reaction) and whole-genome sequencing (reading sequences of the whole DNA present in the sample).

Alleles are different variants (values) of the same gene or the same position (locus), which are located in the same regions (loci) of homologous chromosomes.

A haplotype is a group of alleles that are on a single chromosome and usually correspond to a specific gene. Usually, in a population, there are several stable combinations of genetic variants located with each other on the same chromosome, i.e. haplotypes.

A diplotype is a pair of haplotypes, where each chromosome of a pair of homologous chromosomes has one haplotype (in other words, one haplotype was inherited from one parent, and another was inherited from the other).

A phased diplotype is a diplotype for which it is determined which of the haplotypes was inherited from one parent and which from the other.

An unphased diplotype is a diplotype for which it is not determined which of the haplotypes was inherited from one parent and which from the other.

A homozygous diplotype is a diplotype in which alleles at certain loci are identical to each other on homologous chromosomes, which is equivalent to a pair of identical haplotypes.

A heterozygous diplotype is a diplotype in which alleles at certain loci differ from each other on homologous chromosomes, which is equivalent to a pair of different haplotypes.

A hemizygous diplotype is a diplotype in which there is no homologous allele, i.e. a chromosome with a certain locus does not have a homologous pair, such a diplotype is represented by only one haplotype.

The object of the present invention is to infer a user's phenotype in the absence of complete genetic information.

The proposed method of phenotype inference is based on the fact that the number of possible phenotypes and/or observed variants of molecular effects for all possible diplotypes of a given gene and/or genes is highly limited compared to the number of diplotypes. This invention can be used in different branches of genetics dealing with relations of particular phenotypes with certain variations of nucleotide sequences, including pharmacogenetics and nutrigenetics.

In one embodiment a manifestation of molecular effects depending on a diplotype of one or more genes is the metabolic rate of a substance or substances of a particular type, which is determined by the activity of an enzyme, in other words, by the activity (status) of a metabolizer. In this case, a phenotype may be an observed effect from the use of this substance (a change in the observed parameters, the concentration in the bloodstream, a therapeutic effect, allergic reactions, etc.). Determining the activity of a certain enzyme of a user is a relevant object of pharmacogenetics. Although the present invention relates to inferring any phenotype (and corresponding molecular effects) under conditions of incomplete genetic data, for the convenience of presenting the essence of this invention, a phenotype relating to the metabolism of a drug will be used as an example and the status of its metabolizer as an example of molecular effects.

Other variants of phenotype expression may be different biochemical manifestations, such as the blood level of a particular trace element and/or a metabolite (for example, a vitamin), intolerance to a particular substance (for example, lactose), genetic risk of certain diseases (for example, diabetes mellitus type 2), etc. A user's phenotype inference allows forming personalized recommendations on nutrition, sports, prevention of certain diseases, healthy lifestyle, etc.

If molecular effects are continuous, as for metabolic rate, then their codomain (range of values) can be divided into intervals, groups or categories with obtaining a set of discrete values corresponding to a particular molecular effect (like the metabolizer status in this example). The codomain of a continuous phenotype can be treated similarly. If the codomain of a phenotype and/or molecular effects is discrete, some values can also be combined into groups (categories, clusters) of a higher level.

In one of the embodiments, it is possible to single out several metabolizer statuses caused by variants of the nucleotide sequence of genes encoding enzymes. For example, the following statuses can be singled out: poor, intermediate, normal/extensive, rapid, ultrarapid metabolizers. In some cases, additional statuses can be used, or their number can be reduced (for example, to two: poor and normal metabolizers).

Poor metabolizers are characterized by very low activity (or no activity) of a given enzyme; dysfunction of the corresponding enzyme is typical of this status.

Intermediate metabolizers are characterized by enzyme activity which is below the average.

Normal or extensive metabolizers are characterized by normal or medium activity of a certain enzyme, which is usually the most common.

Rapid metabolizers are characterized by increased enzyme activity.

Ultrarapid metabolizers are characterized by very high enzyme activity.

Additionally, the "unknown metabolizer" status may be used in cases where the enzyme activity is unknown for a known diplotype.

An asterisk with a number and/or a letter may be used to designate a certain gene haplotype. Gene haplotypes may be designated, for example, as *1 or *2A. Then a diplotype will be designated as a pair of haplotypes separated by a slash, for example, *1/*1 (a designation variant for a homozygous diplotype) or *5A/*17 (a designation variant for a heterozygous diplotype).

Some genes may be located on chromosomes that have no homologous pair, then the diplotype will be hemizygous and may be designated as a pair of haplotypes separated by a slash, where the second haplotype is absent, for example, *3/- or *1/0, or as a haplotype constituting this diplotype, for example, *1. The hemizygous state is typical of genes on the X- or Y-chromosome in male subjects or cases of monosomy, such as Turner syndrome in female subjects.

A user's haplotype and/or diplotype may be inferred based on the user's genetic information, which may previously be obtained by genotyping or sequencing a sample of the user's biomaterial (110). The sample may be genotyped by using any known method, for example, by using DNA microchips and a microchip scanner or by using PCR. The sample may be sequenced by Sanger sequencing or by next-generation sequencing (NGS) or by nanopore sequencing or by using other techniques that result in reading a DNA nucleotide sequence.

Data on genotypes, for example, in the form of single nucleotide variations (SNVs), are then obtained from the user's sample by genotyping or sequencing.

The individual genetic information of the user may be presented in the form of position-genotype pairs and/or identifier-genotype pairs. Therewith, the designation of the position and/or identifier of a genetic variation may include the indication of the locus, chromosome and also have indications of the corresponding species and genome assembly version.

For example, it is possible to use the genetic variation designation rsID indicating a genotype, where rsID is the unique identifier of this genetic variation in the database of nucleotide variations dbSNP, supervised by the US National Center for Biotechnological Information. In this case, the haplotype and diplotype will be represented by a plurality of corresponding genetic variations.

Alternatively, it is possible to use genetic variation identifiers using information about the location of a variation in the genome. Such identifiers may include the designation of a chromosome and a position on this chromosome (for example, 7:24926827, where 7 is the chromosome and 24926827 is the position) or designations using HGVS (Human Genome Variation Society) notation with chromosome identifiers, genome assembly versions, positions and nucleotide changes (NC_000007.14:g.24926827C>A, which means the replacement of nucleotide C with A at position 24926827 on chromosome 7 of genome assembly GRCh38).

Any other nomenclatures without limitation may be used to designate the position and/or identifier of a genetic variation. Concurrently, incompleteness of genetic data may be expressed in a lack of information about the user's genotype for one or more genetic variations.

Information about known haplotypes of a gene associated with the phenotype to be inferred may be stored in a database or any other electronic data storage, including distributed data storage. In addition, the data storage may store a description of the molecular effects of each haplotype (including a categorical designation of the efficiency of this gene and/or a description of other effects on the phenotype) as well as information about the frequency of known haplotypes. The frequency of haplotypes may include the population frequency of haplotypes or the frequency in a certain group of subjects, for example, in male subjects, in subjects with a certain diagnosis, etc. In some embodiments of the invention, information about the frequency may imply the population of the entire globe.

In one of the embodiments, it is possible to previously prepare a table describing haplotypes of particular genes and containing information about a set of specific alleles for each gene haplotype (the alleles may be specified in IUPAC nomenclature). For example, in implementing this method, it is possible to use genes of drug-metabolizing enzymes, such as CYP2C9, CYP2D6, CYP2C19, CYP3A5, TPMT and UGT1A1, genes of drug transporters, such as SLCO1B1, ABCB1 and ABCG2, genes of human leukocyte antigen, such as HLA-A and HLA-B, genes of drug targets, such as VKORC1 and CFTR, etc.

Information about known haplotypes of a gene may contain specific alleles that are outside the translated region of the gene if these alleles are of clinical significance. Such alleles can be located in gene introns, thereby disrupting the splicing of its RNA, in enhancer and silencer DNA regions, thereby changing the efficiency of transcription of the gene to which they are specific, as well as in gene regions transcribed to 3' and 5' untranslated RNA regions of a significant impact on the efficiency of its translation and stability or in other DNA regions whose function is currently unclear.

As exemplified by the CYP3A5 gene (cytochrome P450 3A5), information about known haplotypes may look as follows:

**Table 1: Correspondence between the CYP3A5 gene haplotypes and alleles of genetic variations; an empty cell means a match with the reference allele; A, C, G, T are used for adenine, cytosine, guanine, thymine respectively, Y means C or T, and "-" means a deletion.**

| **CYP3A5** | **rs5581 7950** | **rs7767 46** | **rs5596 5422** | **rs5641 1402** | **rs1026 4272** | **rs2838 3479** | **rs4130 3343** | **rs2836 5083** |
|---|---|---|---|---|---|---|---|---|
| ***1** | G | T | A | T | C | C | - | G |
| ***2** | | | | | | | | T |
| ***3** | | C | | | | Y | | |
| ***4** | | | | C | | | | |
| ***5** | | | G | | | | | |
| ***6** | | | | | T | | | |
| ***7** | | | | | | | A | |
| ***8** | A | | | | | | | |
| ***9** | | | | | | T | | |

Information about the frequency of known haplotypes, as exemplified by population frequency in this case, may be presented in the following form:

| **gene** | **haplotype** | **population** | **frequency** |
|---|---|---|---|
| CYP3A5 | *1 | RUS | 0.08067770532056 |
| CYP3A5 | *2 | RUS | 0.00058626572490 |
| CYP3A5 | *3 | RUS | 0.91817549705831 |
| CYP3A5 | *4 | RUS | 0.00000000000000 |
| CYP3A5 | *5 | RUS | 0.00000000000000 |
| CYP3A5 | *6 | RUS | 0.00056053189624 |
| CYP3A5 | *7 | RUS | 0.00000000000000 |
| CYP3A5 | *8 | RUS | 0.00000000000000 |
| CYP3A5 | *9 | RUS | 0.00000000000000 |

A description of the molecular effects of each haplotype may be stored in the following form:

| **CYP3A5 haplotype** | **Molecular effects of the haplotype** |
|---|---|
| *1 | normal function |
| *2 | unknown / undetermined function |
| *3 | no function |
| *4 | unknown / undetermined function |
| *5 | unknown / undetermined function |
| *6 | no function |
| *7 | no function |
| *8 | unknown / undetermined function |
| *9 | unknown / undetermined function |

Specific genetic variations are selected from the user's individual genetic information and then compared with known haplotypes of a particular gene and/or a group of genes, and matching alleles are searched for (120). From the plurality of variations present in the user's individual genotyping data, variations specific to haplotypes of this gene are selected, thus a certain set of variations ***U*** may be obtained from the user's genetic information.

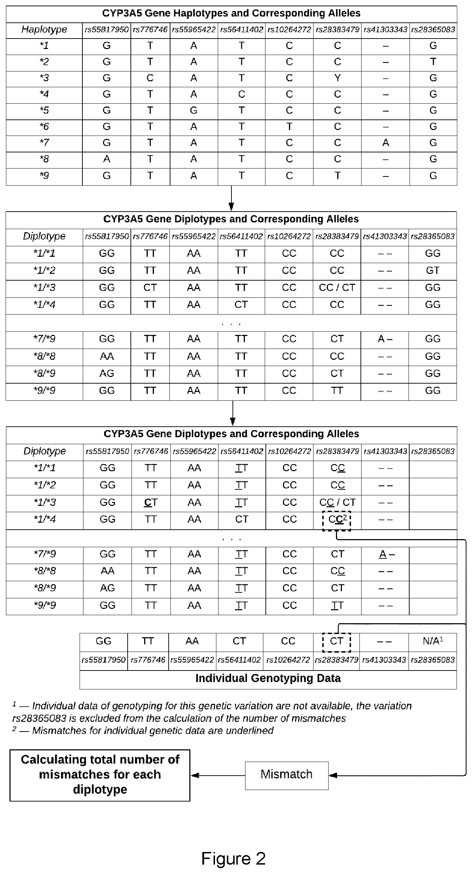

Further, all possible diplotypes of the user for this gene and/or genes are formed (130), as schematically shown for the CYP3A5 gene in Figure 2. A diplotype is a pair of haplotypes for genes on paired chromosomes or a singleton of a haplotype for genes on unpaired chromosomes. Thus, if there are N known haplotypes, then there can be much more possible diplotypes - from N to N². In one of the embodiments, the data storage may also store information about all kinds of diplotypes, which can be formed from known haplotypes of a gene.

Each obtained diplotype variant is then evaluated for compliance with the user's individual genotyping data. For this purpose, the number of genotype mismatches *mᵢ* between the i-th variation of the diplotype and the set of variations U is calculated (140), for example, as shown in Figure 2, and zero mismatches implies a complete match between the sequences.

In one embodiment, if at least one zero mismatch diplotype (m=0) is identified, then all other non-zero mismatch diplotypes may be excluded from further consideration. Concurrently, several variants of zero mismatch diplotypes can be identified due to the incompleteness of genetic data. Selected variants of the diplotypes form a set of D.

In the case where no variant of a complete match diplotype is found, all possible diplotypes may be used for inference.

Alternatively, a certain threshold that excludes one or more diplotypes from further consideration may be applied. For example, a threshold value may be set for the allowable number of mismatches, such a threshold value may be calculated as the median value of the distribution of the number of mismatches *mᵢ,* or in another convenient way. Diplotypes with the number of mismatches satisfying the threshold value form a set of selected diplotype variants D (150).

In another embodiment, based on the comparison between the individual sequence from the set of variations U and possible variants of diplotypes, the minimum number of mismatches *mₘᵢₙ* can be determined from the calculated set of mismatches, and the number k of specific genetic variations with an unknown genotype in the individual data can be calculated. Then the threshold value for the allowable number of mismatches can be defined as the sum of *mₘᵢₙ* and k.

For each diplotype from the selected set of variants ***D***, a probability value is calculated based on data on the frequency of its haplotypes, and such data may be stored in a data storage. In one of the embodiments, the probability value may be calculated based on the normalized value of the frequency of these diplotypes.

In one of the embodiments, the Hardy-Weinberg equation may be used to calculate the frequency of each diplotype on the basis of the frequency of its haplotypes (for example, population frequencies). The frequency of homozygous diplotypes or phased heterozygous diplotypes is the product of the frequencies of haplotypes constituting the diplotype, while this value for unphased heterozygous diplotypes is twice the product of the frequencies of the corresponding haplotypes. The frequency of a hemizygous diplotype will correspond to the frequency of a haplotype by which the diplotype is represented. In this and subsequent illustrative examples, we will use unphased diplotypes, however, it will be obvious to a person skilled in the art that the described method may be implemented relating to phased diplotypes as well.

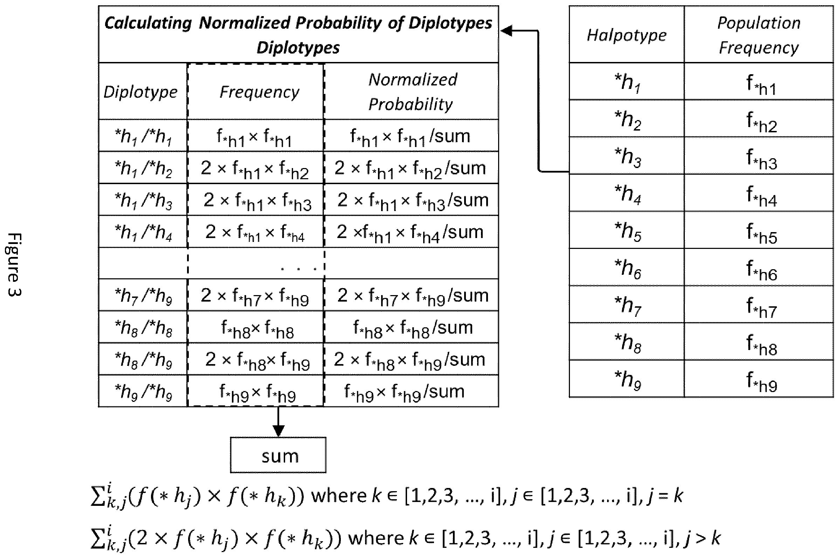

In one of the embodiments, normalized probability values may be used; these probability values are calculated from the frequency of a certain diplotype from the set ***D***, normalized by the sum of all frequencies for selected diplotypes from the set ***D*** (Figure 3). The frequency of a certain diplotype may correspond to the frequency share in a population for this diplotype, the frequency share of the diplotype in subjects of a particular sex or with a particular diagnosis, etc. and may be stored in a data storage.

Further, based on a description of the molecular effects corresponding to a particular haplotype (from the data storage), it is possible to infer the phenotype of each diplotype from the set ***D*** (for example, a categorically described phenotype).

In one of the embodiments, based on a description of the molecular effects of haplotypes, it is possible to determine the molecular effects of each diplotype from the set ***D*** and then to infer the phenotype corresponding to each diplotype on the basis of the molecular effects of the diplotypes.

For some variants of the diplotypes of a particular gene (or a group of genes), molecular effects may be known (for example, the activity of an enzyme encoded or regulated by a given gene and/or genes). Concurrently, some diplotypes of this gene may have unknown molecular effects. For such diplotypes, it is possible to explicitly indicate lack of information and thus formally define an unknown value, for example, as an "unknown metabolizer". As a result, molecular effects can be considered known for each diplotype variant. For all known diplotypes of a particular gene and/or genes, information about their corresponding phenotypes and/or molecular effects may be stored in a data storage.

Furthermore, the number of variants of the molecular effects is much less than the number of all possible diplotypes of a given gene (for example, there may be several hundred diplotypes of a certain gene, while variants of the molecular effects are less than ten), and the number of phenotypes does not exceed and is usually less than the number of molecular effects. Concurrently, at least one mapping that maps diplotypes to their corresponding phenotypes may be defined (160). Particularly, the probability of each phenotype for a user may be calculated based on the probabilities of the corresponding diplotypes. Alternatively, it is possible to define a mapping from diplotypes to molecular effects and then a mapping from these molecular effects to phenotypes. In one of the embodiments, these mappings may be surjective or bijective, and the mappings may be defined by means of a function, a correspondence table or in another convenient way. The mapping defined may be obtained from data storage.

In the described example of the invention, the mapping is defined with a surjective function that completely maps a larger set of diplotypes to a smaller set of phenotypes. The resulting probability of each phenotype may be calculated as the sum of population frequencies, probabilities or normalized probabilities of diplotypes with a given phenotype from the set of variants *D*.

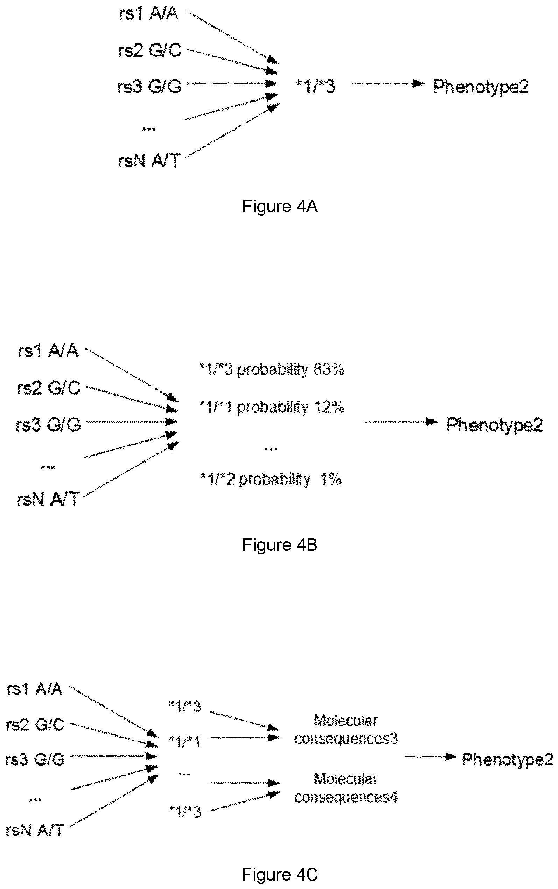

Figure 4A schematically shows how a user's phenotype may be inferred by using a defined surjective mapping, provided that data on his/her genotype are complete. The availability of complete information allows establishing a user's diplotype and the corresponding phenotype uniquely. However, complete genetic data of a user is infrequently available. In addition, even when complete information about genotypes is available, there may be a combination of alleles that does not correspond to any known diplotype. In these cases, the calculation of phenotype values will be probabilistic. The proposed invention enables the most accurate inference of a user's phenotype under conditions of incomplete information about his/her genotypes.

The illustrative embodiment of the invention considers a case where the number of possible haplotypes for a user is not restricted enough. In this case, the user's diplotype cannot be inferred with confidence, and each selected diplotypes will have a certain probability. However, a possible phenotype can be inferred due to the surjective nature of a mapping from a diplotype to a phenotype. A surjective mapping fully maps a domain (or domain of definitions) to a smaller codomain (range), i.e. one or more elements from the domain can be mapped to each element in the codomain.

Figure 4B schematically shows how under conditions of incomplete genetic information of a user, the problem of phenotype inference is solved based on calculating the probability of presence of a particular diplotype by means of a defined surjective mapping in accordance with one embodiment of the invention. In some cases, there may also be a situation where the set of selected diplotypes ***D*** of the user is mapped to a single phenotype. Figure 4B schematically shows how under conditions of incomplete genetic information of a user, the user's phenotype is inferred by means of a sequence (composition) of surjective mappings by intermediately mapping diplotypes to molecular effects and then these molecular effects to phenotypes. Once the user's phenotype has been inferred, the phenotypes may further be mapped to recommendations.

In some embodiments, a mapping from phenotypes to recommendations and/or at least one mapping of a composition of mappings from diplotypes to phenotypes may be bijective. A bijective mapping translates each element from a domain to a single element in a codomain.

In one of the embodiments, it is possible to select a phenotype with the probability value above a certain threshold value, provided that the probability of any other possible phenotypes cannot exceed the specified threshold value. In the case where no phenotype satisfies the specified condition, an artificially assigned value designating an unknown phenotype can be used.

When a user's phenotype is inferred, a personalized recommendation can be formed for this user, for example, a clinically significant recommendation, a recommendation on nutrition, sports, etc. In one of the embodiments, to form recommendations by a user's phenotype, one more mapping from the phenotype to a recommendation may be used, and this mapping may also be surjective or bijective.

In the field of pharmacogenetics, a user's phenotype inference will enable forming recommendations on the use of a particular drug from the analysis of information about genes that influence metabolism, transport, which are involved in the immune response, etc. for this drug. Such recommendations may include recommendations on the drug dosage or a recommendation to dose a drug in accordance with the instructions for medical use or a recommendation to refuse drug administration because of the inefficacy and/or toxicity of said drug for the user or a recommendation to select alternative drugs that will be more effective and/or safer for the user, taking into account the inferred phenotype. These recommendations may be used, for example, by doctors in treatment order and/or in personalized selection of drugs.

A recommendation on a drug may be formed based on known clinical recommendations for different metabolizer statuses that can be found in the public domain. In one of the embodiments, it is possible to use guidelines on the pharmacogenetics of drugs, which are issued by different international medical organizations, in order to form clinically significant recommendations. Such guidelines are issued based on scientific research and contain recommendations for each diplotype in accordance with its molecular effects (metabolizer status in this case).

Thus, as exemplified by the metabolizer of a certain substance, the described approach allows accurately determining the activity level of the corresponding enzyme in a user and/or the probability of each activity level from incomplete genetic data, inferring the user's phenotype from the enzyme activity level and forming personalized recommendations for the user.

Similarly, in the field of nutrigenetics, a user's phenotype inference will enable forming personalized nutritional recommendations, taking into account the individual metabolic characteristics of particular substances and/or predisposition to certain diseases, such as cardiovascular diseases, obesity, diabetes mellitus type 2, etc. Below there are explained examples illustrating the applicability of the described method of phenotype inference for solving technical problems.

As for systems and methods of the present invention, which are aimed at phenotype inference based on incomplete genetic data, these systems and methods may be integrated into or be separated "customer relationship management systems" (CRM systems), for example, medical CRM systems.

In broad terms, such systems are a complex of hardware and software, which includes, for example, server solutions, end-user devices (such as, a personal computer, a portable device connected to the Internet, etc.). Data relating to users (source data) may be uploaded into or downloaded from such a system, for example, via the Internet. This provides a direct start of their automated processing or downloading, for example, recommendations for a user, such as those concerning his/her phenotype, in accordance with the purpose of downloading.

Depending on system modifications and the object, such data may be, for example, uploaded or downloaded directly by a subject or a user and/or a clinical specialist and/or saved to a database (data storage, for example, cloud storage) together with other user data, such as his/her user ID in the database, other information, such as the user's age, sex, etc. The application of the present invention is not limited to a single use for a user, for example, the same means disclosed in the present description enable comparing different user data received with a predetermined time lag (for example, a week, a year, a month, etc.), forming recommendations for the user, including those taking into account the already stored user data, and downloading such data, for example, at the request of the user or a clinical specialist to monitor the success or progress of previously provided recommendations.

Such a system may be implemented, for example, on one or more servers of different types, be a distributed cloud computing system. Access to such a system for its users may be arranged, for example, by creating a user account, for example, by the system administrator, by generating user account data, for example, a login-password pair, or be granted by reading user biometric information (for example, a user's fingerprint readable by means of a specialized cryptographic application on a user terminal or a portable device) or, for example, by means of a smart card, a digital certificate, an electronic digital signature, etc.

Usually, such systems, for example, medical CRM systems, include components such as a front-end part designed to provide services to the system's end-users, such as a doctor, a patient, personnel of a clinic, a medical or health center, a medical laboratory, and to support off-line, centralized or distributed data processing, for example, data of subjects (users) in the context of the present invention; an operating part providing authorization of operations and prompt reporting; a data storage (for example, local or cloud storage) with a distributed (centralized or decentralized) structure; an analysis subsystem (for example, a subsystem configured in the context of the present invention to automatically process data uploaded to this subsystem and to form recommendations for users by their phenotype); a distributed support system providing data replication on endpoints.

Also, such a system may be distributed and have no specific location, which allows lifting the spatial restrictions between its users.

Illustrative examples of the possible use of the proposed method are presented below. These examples are descriptive and are not intended to limit the scope of rights set forth in the claims.

Particularly, as an example of the use of the proposed method in the field of pharmacogenetics, here is described an illustrative example of inferring the phenotype and forming recommendations for a user by means of several surjective mappings, as exemplified by clopidogrel metabolism.

Some drugs are metabolized by liver enzymes (cytochromes). The primary structure of cytochrome proteins is encoded in nuclear DNA genes. Having known the state of some functional alleles in the gene of a specific cytochrome in a particular user, it is possible to establish the optimal dosage of a drug, the probability of side effects or the need to replace it with another drug for this user.

One of the widely used drugs is clopidogrel. It reduces blood clotting ability and is used for the prevention of thrombosis as well as in the treatment of acute myocardial infarction. The active substance is a clopidogrel metabolite formed in the liver under cytochrome 2C19 action, the primary structure of the cytochrome is encoded in the CYP2C19 gene. Normal or high cytochrome activity provides the desired therapeutic effect of clopidogrel, while low or no cytochrome activity leads to ineffective therapy with clopidogrel and requires the selection of another drug independent of cytochrome 2C19. Given the severity of diseases treated with clopidogrel, lack of knowledge about the cytochrome 2C19 activity can lead to fatal consequences due to a lack of therapeutic effect.

The cytochrome 2C19 activity and therefore the phenotype associated with this cytochrome may be inferred by the presence of specific forms of the CYP2C19 gene in a user. In this example, the metabolic rate of clopidogrel (clopidogrel metabolizer status) may be considered as molecular effects. The CYP2C19 gene is located on chromosome 10 and has two copies in a human with a normal karyotype, one inherited from the mother and the other inherited from the father, so one copy may differ from the other. For example, one copy of the gene may encode the enzyme with normal activity, while the other copy may encode the enzyme with high activity. A total of 4 types of cytochrome 2C19 activity are described:
1. high,
2. normal,
3. no,
4. unknown.

Different combinations of the cytochrome activities from each chromosome correspond to different metabolizer statuses, which can be divided into 6 types:
1. Ultrarapid metabolizer (URM) - high + high activities;
2. Rapid metabolizer (RM) - high + normal activities;
3. Normal metabolizer (NM) - normal + normal activities;
4. Intermediate metabolizer (IM) - normal + no or high + no activities;
5. Poor metabolizer (PM) - no + no activities;
6. Unknown metabolizer (?M) - at least one unknown activity.

When using clopidogrel, ultrarapid, rapid and normal metabolizer statuses are beneficial. In cases where the intermediate or poor status of the metabolizer is detected, the doctor may decide to select another drug to replace clopidogrel due to the high probability of ineffective therapy.

The cytochrome activity (and therefore the metabolizer status) is determined by the combination of alleles in the CYP2C19 gene of a particular user. Each gene may have different known stable haplotypes, for example, typical of a certain population, or occurring under other conditions. Table 2 below shows the most frequent haplotypes of the CYP2C19 gene in the European populations as well as allele states that correspond to these haplotypes. For example, variation g.85186A>G means that there is allele A at the corresponding position in the human reference genome, but people from the population have allele G at this position, i.e. the reference allele A was replaced with the alternative allele G, which is designated as A>G.

**Table 2: Correspondence between the CYP2C19 gene haplotypes and alleles of genetic variations; an empty cell means a match with the reference allele; A, C, G, T are used for adenine, cytosine, guanine, thymine respectively, R means A or G, Y means C or T, "-" means a deletion; a genetic variation is a known genetic variation at a specific position of the reference sequence NG_008384.3 (reference sequence identifier of the CYP2C19 gene in the RefSeq database).**

| **Genetic variation** | **Reference allele** | **Haplotypes** | | | | | | | | | | | | |
|---|---|---|---|---|---|---|---|---|---|---|---|---|---|---|
| | | ***1** | ***2** | ***3** | ***4** | ***5** | ***6** | ***7** | ***8** | ***9** | ***13** | ***14** | ***15** | ***17** |
| g.85186A>G | A | R | G | G | G | R | G | - | - | G | G | G | G | G |
| g.17687A>G | A | | G | | | | | | | | | | | |
| g.24179G>A | G | | A | | | | | | | | | | | |
| g.22973G>A | G | | | A | | | | | | | | | | |
| g.5026A>G | A | | | | G | | | | | | | | | |
| g.95058C>T | C | | | | | T | | | | | | | | |
| g.17773G>A | G | | | | | | A | | | | | | | |
| g.24319T>A | T | | | | | | | A | | | | | | |
| g.17736T>C | T | | | | | | | | C | | | | | |
| g.17809G>A | G | | | | | | | | | A | | | | |
| g.92315C>T | C | | | | | | | | | | T | | | |
| g.5075T>C | T | | | | | | | | | | | C | | |
| g.5080A>C | A | | | | | | | | | | | | C | |
| g.4220C>T | C | | | | Y | | | | | | | | | T |

Table 3 shows the activity type of cytochrome 2C19, which corresponds to each haplotype, as well as the frequency of these haplotypes in the European populations.

**Table 3: Frequency of the CYP2C19 gene haplotypes in the European populations and correspondence between the haplotype and cytochrome 2C 19 activity.**

| **CYP2C19 gene haplotype** | **Frequency in the population** | **Cytochrome 2C19 activity** |
|---|---|---|
| *1 | 75.4733% | Normal |
| *2 | 14.2594% | No |
| *3 | 0.1513% | No |
| *4 | 0.021 % | No |
| *5 | 0.0014% | No |
| *6 | 0.0057% | No |
| *7 | 0.0000% | No |
| *8 | 0.0651 % | No |
| *9 | 0.0029% | Low |
| *13 | 0.0087% | Normal |
| *14 | 0.0009% | Unknown |
| *15 | 0.0007% | Normal |
| *17 | 10.0095% | High |

For example, the haplotype designated as *1 corresponds to the following combination of allele states in the CYP2C19 gene:

| **Genetic variation** | **Allele** |
|---|---|
| g.85186A>G | A |
| g.17687A>G | A |
| g.24179G>A | G |
| g.22973G>A | G |
| g.5026A>G | A |
| g.95058C>T | C |
| g.17773G>A | G |
| g.24319T>A | T |
| g.17736T>C | T |
| g.17809G>A | G |
| g.24178C>T | C |
| g.17827G>A | G |
| g.95234A>C | A |
| g.92315C>T | C |
| g.5075T>C | T |
| g.5080A>C | A |
| g.95085C>T | C |
| g.4220C>T | C |

If the above combination occurs in one of the 10th chromosomes of any person, we can say that on this chromosome, he/she has a form of the CYP2C19 gene, which is designated as *1.

A pair of the CYP2C19 gene copies corresponds to a diplotype (a pair of haplotypes), which is designated as *1/*1 for cases where both copies of the gene correspond to the form *1. The diplotype *1/*1 corresponding to the metabolizer status NM will be observed in a person with the following set of genotypes:

| **Genetic variation** | **Genotype** |
|---|---|
| g.85186A>G | A/A |
| g.17687A>G | A/A |
| g.24179G>A | G/G |
| g.22973G>A | G/G |
| g.5026A>G | A/A |
| g.95058C>T | C/C |
| g.17773G>A | G/G |
| g.24319T>A | T/T |
| g.17736T>C | T/T |
| g.17809G>A | G/G |
| g.24178C>T | C/C |
| g.17827G>A | G/G |
| g.95234A>C | A/A |
| g.92315C>T | C/C |
| g.5075T>C | T/T |
| g.5080A>C | A/A |
| g.95085C>T | C/C |
| g.4220C>T | C/C |

Using Table 2, it is possible to infer a combination of genotypes, which corresponds to any pair of haplotypes. Table 4 provides several examples of diplotypes and their corresponding genotypes.

**Table 4: Combinations of genotypes of genetic variations in the CYP2C19 gene, corresponding to diplotypes *8/*17 and *1/*3.**

| **Genetic variation** | **Genotypes** | |
|---|---|---|
| g.85186A>G | A/A | A/A |
| g.17687A>G | A/A | A/A |
| g.24179G>A | G/G | G/G |
| g.22973G>A | G/G | G/A |
| g.5026A>G | A/A | A/A |
| g.95058C>T | C/C | C/C |
| g.17773G>A | G/G | G/G |
| g.24319T>A | T/T | T/T |
| g.17736T>C | C/T | C/T |
| g.17809G>A | G/G | G/G |
| g.92315C>T | C/C | C/C |
| g.5075T>C | T/T | T/T |
| g.5080A>C | A/A | A/A |
| g.4220C>T | C/T | C/T |
| | | |
| Diplotype | *8/*17 | *1/*3 |

To infer the diplotype and then the metabolizer status, it is necessary to sequence the CYP2C19 gene or to genotype its key genetic variations. Although accurate, this process is not always successful, and some gene positions may lack information about the genotypes. Conventional algorithms (for example, a greedy algorithm) require information about each gene position to infer a diplotype; in such a case, further interpretation will be impossible due to incomplete input data. In broad terms, a greedy algorithm consists of making locally optimal decisions at each stage, while assuming that the final solution will also be optimal (for example, Huffman, Kruskal, Prim, and other algorithms).

In some cases, this technical disclosure allows an unambiguous interpretation under conditions of incomplete genetic information. If a missing unit of information (a missing genotype) in the set of data (genotypes) is substituted with all its possible values (superposition), then all potential possible interpretations can be obtained. Possible values for a particular genetic variation may be taken from Table 2. It is further illustrated, how a lack of information about the genotype of only one position increases the number of possible diplotypes.

**Table 5: Influence of lack of information about the variation g. 17687A>G genotype on an increase in the number of possible diplotypes.**

| **Genetic variation** | **User's genotype** | **Possible genotypes of variation g.17687A>G** | | |
|---|---|---|---|---|
| g.85186A>G | G/G | G/G | G/G | G/G |
| **g.17687A>G** | **?/?** | **A/A** | **A/G** | **G/G** |
| g.24179G>A | G/G | G/G | G/G | G/G |
| g.22973G>A | G/G | G/A | G/A | G/A |
| g.5026A>G | A/A | A/A | A/A | A/A |
| g.95058C>T | C/C | C/C | C/C | C/C |
| g.17773G>A | G/G | G/G | G/G | G/G |
| g.24319T>A | T/T | T/T | T/T | T/T |
| g.17736T>C | C/T | C/T | C/T | C/T |
| g.17809G>A | G/G | G/G | G/G | G/G |
| g.92315C>T | C/C | C/C | C/C | C/C |
| g.5075T>C | T/T | T/T | T/T | T/T |
| g.5080A>C | A/A | A/A | A/A | A/A |
| g.4220C>T | C/T | C/T | C/T | C/T |
| | | | | |
| Possible diplotypes | | *1/*1 | *1/*2 | *2/*2 |

The greater the number of information units the data set lacks, the greater the number of possible diplotypes that can correspond to this information set. Let's consider a certain user in whose set of genotypes, three significant positions lack information, as an illustrative example of the application of the above method of phenotype inference based on incomplete genetic information.

**Table 6: Set of genotype variations of the CYP2C19 gene, where there is no information about the state of variations g.92315C>T, g.5080A>C and g.4220C>T.**

| **Genetic variation** | **User's genotype** |
|---|---|
| g.85186A>G | G/G |
| g.17687A>G | A/A |
| g.24179G>A | G/G |
| g.22973G>A | G/G |
| g.5026A>G | A/A |
| g.95058C>T | C/C |
| g.17773G>A | G/G |
| g.24319T>A | T/T |
| g.17736T>C | C/T |
| g.17809G>A | G/G |
| **g.92315C>T** | **?/?** |
| g.5075T>C | T/T |
| **g.5080A>C** | **?/?** |
| **g.4220C>T** | **?/?** |

If we consider all possible values of the missing genotypes, the resulting combinations (27 possible combinations) can be mapped to the following diplotypes in accordance with the correspondences from Table 2:
- *1/*1
- *1/*13
- *1/*15
- *1/*17
- *13/*13
- *13/*15
- *13/*17
- *15/*15
- *15/*17
- *17/*17

The selected diplotypes completely match with the known genotypes of the user, i.e. the number of mismatches for these variants is zero. Each diplotype may be mapped to one out of the 6 metabolizer statuses and has a certain frequency. In one of the embodiments, the frequency of each diplotype may be calculated from the frequency of its haplotypes by using, for example, the Hardy-Weinberg equation. Or the frequency may be retrieved directly from data storage. It is also possible to calculate the normalized probability values of these diplotypes, as described above. In this case, the previously obtained possible diplotypes of the user are mapped to 3 metabolizer statuses out of possible 6: Normal (77.96%), Rapid (20.67%) and Ultrarapid (1.37%), as shown in Table 8.

**Table 7: Correspondence between the CYP2C19 gene diplotype and metabolizer status by cytochrome 2C19, frequency of each diplotype and normalized probability of identification.**

| **Diplotype** | **Cytochrome 2C19 metabolizer status** | **Frequency** | **Normalized identification probability** |
|---|---|---|---|
| *1/*1 | Normal | 56.96% | 77.94% |
| *1/*13 | Normal | 0.013% | 0.018% |
| *1/*15 | Normal | 0.0011% | 0.0015% |
| *1/*17 | Rapid | 15.11% | 20.67% |
| *13/*13 | Normal | 0.000001% | 0.0000014% |
| *13/*15 | Normal | 0.0000001% | 0.00000014% |
| *13/*17 | Rapid | 0.0017% | 0.0023% |
| *15/*15 | Normal | 0.0000000049% | 0.00000001 % |
| *15/*17 | Rapid | 0.00014% | 0.00019% |
| *17/*17 | Ultrarapid | 1% | 1.37% |

All three variants are beneficial for clopidogrel. This makes it possible to infer the phenotype expressed in the desired therapeutic effect of clopidogrel with a probability of 100% and form a recommendation to use this drug in accordance with the instructions for medical use.

Thus, even with incomplete genetic information of a user, it is possible to draw an unambiguous conclusion about the therapeutic effect of a drug. This is enabled by sequential surjective mapping from genotypes to a recommendation. In one of the embodiments, a sequential surjective mapping may be done by one or more surjective functions restricting a larger set of diplotypes to a smaller set of phenotypes and then to recommendations:
1. the 27 possible combinations of genotypes are mapped to 10 possible diplotypes;
2. the 10 diplotypes are mapped to 3 possible metabolizer statuses;
3. the 3 metabolizer statuses are mapped to 1 possible recommendation on prescribing clopidogrel.

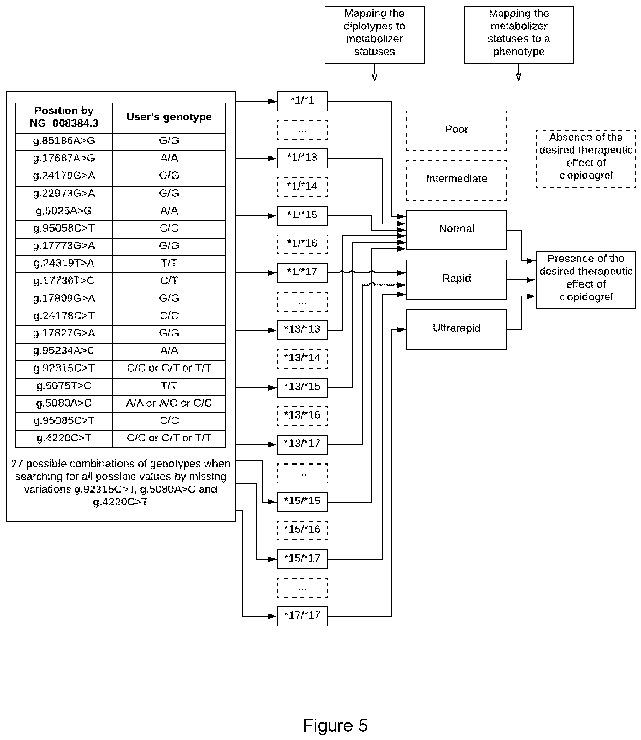

Figure 5 shows a diagram illustrating the proposed method of surjective mapping, as exemplified by clopidogrel metabolism. The diagram depicts a composition of two surjective mappings that sequentially map diplotypes to metabolizer statuses (i.e. molecular effects) and then these metabolizer statuses to a phenotype. The dashed line shows elements of the codomain, to which genotype data were not mapped. An ellipsis designates multiple groups of elements from the codomain, located between the nearest values indicated in the picture - this designation is introduced to simplify the diagram.

Additionally, one more mapping from a phenotype to recommendations may be used. This mapping may be bijective (when there is a corresponding recommendation for each phenotype) or surjective (if one recommendation may correspond to several phenotypes).

As another embodiment, which does not also limit the scope of the proposed invention and illustrates an application of this invention in the field of nutrigenetics, a sequential surjective mapping is presented here, which allows inferring a user's phenotype and providing appropriate recommendations from the inferred blood vitamin D level. In general terms, this example illustrates a fundamentally different use of the described approach to phenotype inference. When a phenotype depends on several genes and each gene contributes unequally, it is possible to determine their total influence by using weighting factors. Such weighting factors may be selected in advance, for example, in the course of research, or be determined based on scientific publications. Under conditions of incomplete genetic data, a range of possible values for a weighted sum can be evaluated, therewith different values of the weighted sum may be mapped to one phenotype since the number of phenotypes for a certain factor is very limited.

Blood vitamin D level is a complex trait depending on many factors, and genetic factors are among those that contribute a lot. Transfer proteins, enzymes responsible for the absorption of vitamin D from the intestine into the blood and for its metabolism into active forms, specific receptors through which active forms of vitamin D act on cells
- all these protein molecules are encoded in human genes. The major genes include:
   - GC - encodes a transfer protein that circulates in the blood plasma, intercellular, cerebrospinal fluids and transports vitamin D and its metabolites to cells and tissues;
   - CYP2R1 - encodes a hepatic enzyme of the cytochrome P450 system, which catalyzes the reaction of converting vitamin D into its active metabolite being a ligand to vitamin D receptors;
   - VDR - encodes a receptor for the active form of vitamin D and mediates the action of this vitamin on human cells.

Different variants of the nucleotide sequence of these genes may influence the functions of the encoded proteins: change the receptor or transporter affinity for vitamin D or change the enzyme activity. Since different variants of these genes present in the population, different people have different rates of vitamin D absorption and metabolism, and this ultimately influences its concentration in the bloodstream. Knowing genetic variations associated with vitamin D levels, it is possible to assess its level from human genetic data.

At the present day, it is undoubtedly believed that specific alleles are associated with blood vitamin D levels to a different extent (refer, for example, [1] Jiang, X. et al., "Genome-wide association study in 79,366 European-ancestry individuals informs the genetic architecture of 25-hydroxyvitamin D levels", Nature Communications, 9, (2018); [2] Dastani, Z. et al., "Genetic Regulation of Vitamin D Levels", Calcified Tissue International, 92, 2, (2012)). Table 8 shows the extent to which associated genetic variants influence the level.

**Table 8: Genetic variations associated with blood vitamin D levels. An effector allele is an allele associated with changes in the vitamin D level; an effect is the amount by which the vitamin D level changes in owners of the effector allele; RefSeq is an identifier of the reference nucleotide sequence of the gene in the RefSeq database.**

| Gene | RefSeq | Genetic variation | Effector allele | Effect |
|---|---|---|---|---|
| GC | NG_012837.3 | g.66840G>T | G | 0.23 |
| CYP2R1 | NG_007936.1 | g.3874T>C | T | -0.39 |
| VDR | NG_008731.1 | g.30920T>A | A | -0.12 |

Blood vitamin D level may be considered as one of the phenotype variants in the context of the present invention. One possible model for predicting the vitamin D level from the presented data is a weighted sum of a user's effector alleles in conjunction with one or more thresholds to which this sum is compared, therewith the user is classified as an owner of one phenotype out of three - high, medium or low vitamin D levels. The weighted sum may be calculated by the following formula: w = 0,23 · x + (-0,39) · *y* + (-0,12) · z, where x is the number of alleles G in the user's genotype for genetic variation g.66840G>T; y is the number of alleles T in the user's genotype for genetic variation g.3874T>C; z is the number of alleles A in the user's genotype for genetic variation g.30920T>A. For the convenience of presenting the essence of this invention, let's consider an example when 2 threshold values -0.12 and 0.12 are set. Then the classification criteria will be as follows:
- if the weighted sum w < -0.12, then the user will be assigned to the Low Vitamin D Level phenotype,
- if -0.12 ≤ w < 0.12, then the user will be assigned to the Medium Vitamin D Level phenotype, and
- if w ≥ 0.12, then the user will be assigned to the High Vitamin D Level phenotype.

Depending on the user's phenotype, different recommendations may be issued. For the low level, it may be recommended to include food products rich in vitamin D in the diet, while for the other two phenotypes, there may not be such a recommendation.

For example, for the model presented, a user with the following genotypes, as shown in Table 9, will have a weighted sum of 0.07 and will fall into the Medium Vitamin D Level group and will not receive a recommendation to change the diet:

**Table 9: Genotypes of a virtual user who is classified as an owner of a medium blood vitamin D level.**

| Genetic variation | Genotype |
|---|---|
| g.66840G>T | G/G |
| g.3874T>C | C/T |
| g.30920T>A | T/T |

When data on the genotypes of all necessary genetic variations are available, it is a trivial task to give an interpretation and a recommendation for a user. The proposed method of phenotype inference allows interpreting a user's genetic information even in the absence of certain genotypes and achieving the same result.

In the example below, a user's genotype of variation g.66840G>T is unknown. If it is substituted with all possible values, then a weighted sum can be calculated:

**Table 10: User's genotypes without data on variation g.66840G>T and possible values of the weighted sum resulted from the search of all possible values of the unknown variation.**

| Genetic variation | Genotype | Possible genotypes of variation g.66840G>T | | |
|---|---|---|---|---|
| g.66840G>T | ?/? | T/T | G/T | G/G |
| g.3874T>C | C/C | C/C | C/C | C/C |
| g.30920T>A | A/T | A/T | A/T | A/T |
| | | | | |
| Possible weighted sum | | -0.12 | +0.11 | +0.34 |

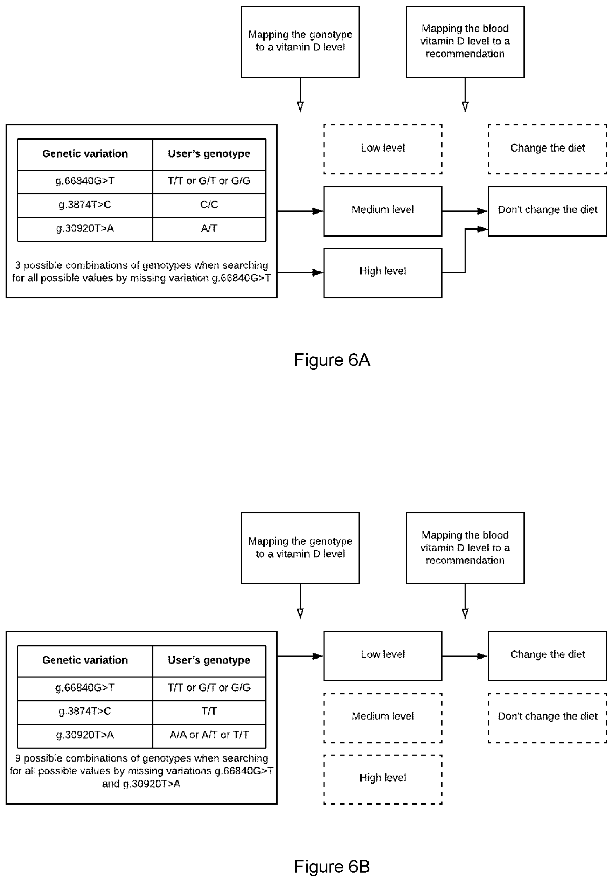

All three possible values of the weighted sum do not fall into the Low Vitamin D Level group, so even when there is no value of variant g.66840G>T, this user may definitely not be provided with a recommendation to change the diet. Figure 6A shows a diagram illustrating the proposed method of surjective mapping, as exemplified by vitamin D levels.

An unambiguous result can be obtained even in some cases where a lot of data is not available. For example, the following user lacks information about the genotypes of two genetic variations.

**Table 11: User's genotypes without data on variations g.66840G>Tand g.30920T>A and possible values of the weighted sum resulted from the search of all possible combinations of the unknown variations.**

| Genetic variation | Genotype | Possible genotype combinations of variations g.66840G>T and g.30920T>A | | | | | | | | |
|---|---|---|---|---|---|---|---|---|---|---|
| g.66840G>T | ?/? | T/T | G/T | G/G | T/T | G/T | G/G | T/T | G/T | G/G |
| g.3874T>C | T/T | T/T | T/T | T/T | T/T | T/T | T/T | T/T | T/T | T/T |
| g.30920T>A | ?/? | A/A | A/A | A/A | A/T | A/T | A/T | T/T | T/T | T/T |
| | | | | | | | | | | |
| Weighted sum | | -1.02 | -0.79 | -0.56 | -0.9 | -0.67 | -0.44 | -0.78 | -0.55 | -0.32 |

In this case, the value of the weighted sum for any combination of possible genotypes will be below the first threshold value (-0.12) and definitely falls into the Low Vitamin D Level group, and this user will receive a recommendation to include food products rich in vitamin D in the diet. Figure 6B shows a diagram illustrating this example.

Thus, the described method of phenotype inference is based on using the property of surjective mappings to completely map a domain of a given size to a codomain of a smaller size. In some cases, this allows a mapping from several variants to a single value by means of one or more surjective mappings, for example, in cases where the input is a superposition of several possible genotypes (all possible combinations of states taking into account missing information units).

In addition to the method described above, the proposed invention is aimed at a computer system that allows the proposed method to be implemented as well as at a computer-readable medium. It is also claimed a computer system that provides processing in a computing environment, contains a memory and a processor interacting with the memory and is configured to implement the proposed method of a user's phenotype inference in the absence of complete genetic information. Similarly, the invention relates to a computer-readable data medium that is configured to be read by a data processing device and stores commands to be executed by a data processing device for implementing the proposed method of a user's phenotype inference in the absence of complete genetic information.

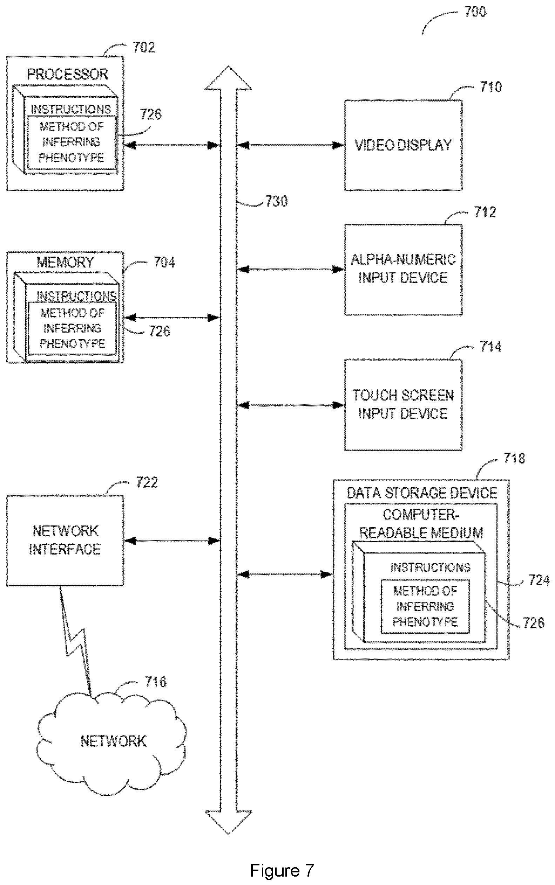

Figure 7 shows a component diagram for an example of a computing system that may be used to implement the method described herein of a user's phenotype inference in the absence of complete genetic information. The computing system 700 may be connected to another computing system via a local area network, a corporate network, an extranet or the Internet. The computing system 700 may operate as a server or a client in a client-server network environment or as a peer-to-peer computing device in a peer-to-peer (or distributed) network environment. The computing system 700 may be represented by a personal computer (PC), a tablet computer, a set-top box (STB), a pocket computer (PDA), a cell phone or any computing system capable of (sequentially or otherwise) executing a set of commands that determine the operations to be performed by this computing system. Moreover, although only one computing system is shown, the term "computing system" may also include any group of computing systems that separately or together execute a set (or multiple sets) of commands to perform one or more techniques discussed herein.

An example of the computing system 700 includes a processor 702, a main memory 704 (for example, read-only memory (ROM) or dynamic random-access memory (DRAM)) and a storage device 718, which communicate with each other over a bus 730.

The processor 702 may be represented by one or more general-purpose computing devices, such as a microprocessor, a central processing unit, etc. Particularly, the processor 702 may be a complete instruction set computer (CISC), a reduced instruction set computer (RISC), a very long control word processor (VLIW) or a processor in which other sets of commands are implemented or processors in which a combination of command sets is implemented. The processor 702 may also be one or more special-purpose computing devices, such as an application-specific integrated circuit (ASIC), a field-programmable gate array (FPGA), a digital signal processor (DSP), a network processor, etc. The processor 702 is arranged to execute commands 726 to implement the methods discussed herein.

The computing system 700 may additionally include a network interface device 722, a visual display unit 710, a character input device 712 (for example, a keyboard), and an input device represented by a touch screen 714.

The storage device 718 may include a computer-readable data medium 724, which stores one or more sets of commands 726 in which one or more methods or functions described in this embodiment are implemented. During the execution of commands 726 in the computing system 700, they may also be in the main memory 704 and/or in the processor 702 completely or at least partially, therewith the main memory 704 and the processor 702 are also computer-readable data media. Commands 726 may also be transmitted or received via a network 716 through the network interface device 722.

In some embodiments, commands 726 may include commands for inferring a user's phenotype in the absence of complete genetic information, in accordance with one or more embodiments of the present invention. Although the computer-readable data medium 724, shown in the example in Figure 7, is a single medium, the term "computer-readable medium" may include one or more media (for example, a centralized or distributed database and/or corresponding caches and servers) that store one or more sets of commands. The term "computer-readable data medium" may also include any medium that can store, encode or contain a set of commands to be executed by a computer and that enables a computer to execute any one or more techniques of the present invention. Therefore, the term "computer-readable data medium" refers to, among other things, solid-state storage devices as well as optical and magnetic media.

The methods, components, and functions described herein may be implemented by means of discrete hardware components or may be integrated into functions of other hardware components, such as an application-specific integrated circuit (ASIC), a field-programmable gate array (FPGA), a digital signal processor (DSP) or similar devices. In addition, the methods, components, and functions may be implemented by means of embedded software modules or hardware function charts. The methods, components, and functions may also be implemented by means of any combination of hardware and software components or by means of software exclusively.

The above description sets forth numerous details. However, it should be obvious to any person skilled in the art, who has read this description, that in practice, the present invention can be implemented without these specific details. In some cases, well-known structures and devices are shown in the form of block diagrams without going into details so as not to complicate the description of the present invention.

Some portions of the description of the preferred embodiments are presented in the form of algorithms and symbolic representations of operations with data bits within a computer memory device. These algorithmic descriptions and representations are means employed by specialists in the field of data processing to most effectively convey the substance of their work to others skilled in the art. In the context of this description, as is customary, an algorithm is a logically consistent sequence of operations leading to the desired result. The operations involve actions requiring physical manipulations of physical quantities. Typically, though not necessarily, these quantities take the form of electrical or magnetic signals capable of being stored, transmitted, combined, compared and otherwise manipulated. It is convenient at times, principally for reasons of common usage, to describe these signals as bits, values, elements, symbols, terms, numbers, etc.

However, it should be borne in mind that all of these and similar terms are to be associated with the appropriate physical quantities and are merely convenient designations applied to these quantities. Unless further specified, it is accepted that in the following description, the terms "determination," "calculation," "computation," "obtaining," "establishment," "change", etc. relate to actions and processes of the computing system or a similar electronic computing system that uses and transforms data represented as physical (for example, electronic) quantities in the registers and memory devices of the computing system into other data that are similarly represented as physical quantities in the memory devices or registers of the computing system or other devices for storing, transmitting or displaying such information.

The present invention also relates to a device for performing the operations described herein. Such a device may be specially designed for the required purposes or may be a general-purpose computer that is selectively actuated or further configured by a program stored in the computer's memory. Such a computer program may be stored on a computer-readable data medium, such as, but not limited to, disks of any type, including floppy disks, optical disks, CD-ROMs and magnetic optical disks, read-only memory (ROM), random-access memory (RAM), EPROM, EEPROM, magnetic or optical cards and any type media suitable for storing electronic information.

The computer program code of one or more computer programs for performing the operations to implement the steps (implementation stages) of this technical disclosure may be written in any programming language or a combination of programming languages, including an object-oriented programming language, such as Python, R, Java, Smalltalk, C++, etc., and conventional procedural programming languages, such as the programming language "C", functional programming languages, such as Haskell, Agda, F#, Formality, or similar programming languages, not limited to combinations of languages for many different parts of the program code. The program code may be executed on a user's computer in full, in part or as a separate software package, partly on a user's computer and partly on a remote computer, or completely on a remote computer, a server or other computer device comprising a processor and a memory interconnected with each other. In the latter case, the remote computer may be connected to the user's computer via any type network, including a local area network (LAN), a wide area network (WAN) or a connection to an external computer (for example, via the Internet through internet-service providers).

It will be appreciated that the above description is intended to illustrate rather than limit the essence of the invention. After reading and understanding the above description, other different embodiments of the invention will become apparent to those skilled in the art. On this basis, the application of the invention should be determined taking into account the appended claims as well as all applications of equivalent methods, which are equally covered by the claims.

## Claims

1. A computer-implemented method of a user's phenotype inference in the absence of complete genetic information, the method comprising:
obtaining a user's genetic information with genetic variations in a form of position-genotype pairs and/or identifier-genotype pairs, and an incompleteness of the user's genetic information is reflected in an unknown genotype for at least one position and/or identifier of a genetic variation,
selecting a set of genetic variations from the user's genetic information including at least one variation with unknown genotype, which are specific for known haplotypes of at least one gene associated with the phenotype, wherein the known haplotypes are obtained from a data storage,
obtaining all possible diplotypes from known haplotypes for said at least one gene associated with the phenotype,
substituting all possible values of at least one variation with unknown genotype from the set of genetic variations,
evaluating each obtained diplotype for similarity with the obtained user's genetic information by calculating a number of mismatches between the diplotype and the set of genetic variations,
forming at least one set of diplotypes D for at least one gene associated with the phenotype based on the calculated number of mismatches, and
inferring the user's phenotype by using the set of diplotypes D and a mapping from diplotypes of said at least one gene to phenotypes obtained from the data storage.

2. The method of a user's phenotype inference of claim 1, wherein the data storage additionally stores information about a frequency of known haplotypes.

3. The method of a user's phenotype inference of claim 1, wherein the data storage additionally stores a description of molecular effects of each known haplotype for said at least one gene associated with the phenotype.

4. The method of a user's phenotype inference of claim 3, further comprising:
inferring molecular effects of at least one diplotype for said at least one gene associated with the phenotype based on a description of molecular effects of at least one haplotype constituting this diplotype, and
mapping from at least one diplotype to a phenotype is calculated based on the description of the molecular effects of this diplotype.

5. The method of a user's phenotype inference of claim 1, wherein forming at least one set of diplotypes D includes all possible diplotypes if no variant of the diplotype with a complete match with the set of genetic variations is found when evaluating each obtained diplotype.

6. The method of a user's phenotype inference of claim 1, wherein evaluating each obtained diplotype further comprises:
applying at least one threshold value to the number of mismatches, which excludes one or more diplotypes from further consideration when.

7. The method of a user's phenotype inference of claim 2, wherein for each diplotype from the set D, a probability value is calculated based on data on its haplotype frequency, and the probability value is equal to the product of the frequencies of haplotypes constituting the diplotype for homozygous diplotypes and for phased heterozygous diplotypes or twice the product of the frequencies of haplotypes constituting the diplotype for unphased heterozygous diplotypes or the value of the frequency of a constituent haplotype for hemizygous diplotypes.

8. The method of a user's phenotype inference of claim 1 further comprises:
calculating the probability of each phenotype value for the user.

9. The method of a user's phenotype inference of claim 8, wherein the mapping is presented by at least one surjective mapping that completely maps a larger set of diplotypes to a smaller set of phenotypes, and the probability of each phenotype for the user may be calculated as the sum of the previously obtained probabilities of the corresponding diplotypes from the set D.

10. The method of a user's phenotype inference of claim 1 further comprises:
forming one or more recommendations for the user, taking into account the inferred phenotype.

11. The method of a user's phenotype inference of claim 1, wherein at least one gene associated with the phenotype belongs to a group of genes of drug-metabolizing enzymes or genes of drug transporters or genes of human leukocyte antigen or genes of drug targets.

12. The method of a user's phenotype inference of claim 1, wherein all possible diplotypes are obtained by extracting data on the diplotypes from a data storage.

13. A system for inferring a user's phenotype in the absence of complete genetic information, which comprises a data processing device and a memory operatively linked to the processing device, and this system is configured for:
obtaining a user's genetic information with genetic variations in a form of position-genotype pairs and/or identifier-genotype pairs, and an incompleteness of the user's genetic information is reflected in an unknown of a genotype for at least one position and/or identifier of a genetic variation,
selecting a set of genetic variations from the user's genetic information including at least one variation with unknown genotype, which are specific for known haplotypes of at least one gene associated with the phenotype, wherein the known haplotypes are obtained from a data storage,
obtaining all possible diplotypes from known haplotypes for said at least one gene associated with the phenotype,
substituting all possible values of at least one variation with unknown genotype from the set of genetic variations,
evaluating each obtained diplotype for similarity with the obtained user's genetic information by calculating a number of mismatches between a diplotype and the set of genetic variations,
forming at least one set of diplotypes D for at least one gene associated with the phenotype based on the calculated number of mismatches, and
inferring the user's phenotype by using the set of diplotypes D and a mapping from diplotypes of said at least one gene to phenotypes obtained from the data storage.

14. The system for inferring a user's phenotype of claim 18, which is additionally configured for forming one or more recommendations for the user, taking into account the inferred phenotype.

15. A computer-readable data medium that is configured to be read by a data processing device and stores commands to be executed by a data processing device for inferring a user's phenotype in the absence of complete genetic information, and said commands, when executed by the data processing device, induce it to execute:
obtaining a user's genetic information with genetic variations in a form of position-genotype pairs and/or identifier-genotype pairs, and a incompleteness of the user's genetic information is reflected in an unknown of a genotype for at least one position and/or identifier of a genetic variation,
selecting a set of genetic variations from the user's genetic information including at least one variation with unknown genotype, which are specific for known haplotypes of at least one gene associated with the phenotype, wherein the known haplotypes are obtained from a data storage,
obtaining all possible diplotypes from known haplotypes for said at least one gene associated with the phenotype,
substituting all possible values of at least one variation with unknown genotype from the set of genetic variations,
evaluating each obtained diplotype for similarity with the obtained user's genetic information by calculating a number of mismatches between a diplotype and the set of genetic variations,
forming at least one set of diplotypes D for at least one gene associated with the phenotype based on the calculated number of mismatches, and
inferring the user's phenotype by using the set of diplotypes D and a mapping from diplotypes of said at least one gene to phenotypes obtained from the data storage.
